(19) Europäisches Patentamt
European Patent Office
Office européen des brevets

(11) **EP 2 890 360 B1**

(12) **FASCICULE DE BREVET EUROPEEN**

(45) Date de publication et mention de la délivrance du brevet:
**29.09.2021 Bulletin 2021/39**

(21) Numéro de dépôt: **13779124.0**

(22) Date de dépôt: **29.08.2013**

(51) Int Cl.:
*A61K 8/02* $^{(2006.01)}$  *A61K 8/67* $^{(2006.01)}$
*A61K 8/73* $^{(2006.01)}$  *A61L 27/02* $^{(2006.01)}$
*A61L 27/20* $^{(2006.01)}$  *A61L 27/52* $^{(2006.01)}$
*A61L 27/54* $^{(2006.01)}$  *A61Q 19/08* $^{(2006.01)}$
*C08B 37/08* $^{(2006.01)}$  *C08K 5/41* $^{(2006.01)}$
*C08K 5/529* $^{(2006.01)}$  *C08L 5/08* $^{(2006.01)}$
*A61K 8/44* $^{(2006.01)}$

(86) Numéro de dépôt international:
**PCT/EP2013/002597**

(87) Numéro de publication internationale:
**WO 2014/032804 (06.03.2014 Gazette 2014/10)**

(54) **COMPOSITION, STERILISEE, COMPRENANT AU MOINS UN ACIDE HYALURONIQUE ET DE L'ASCORBYL PHOSPHATE DE MAGNESIUM**

STERILISIERTE ZUSAMMENSETZUNG MIT MINDESTENS EINER HYALURONSÄURE UND MAGNESIUMASCORBYLPHOSPHAT

STERILISED COMPOSITION COMPRISING AT LEAST ONE HYALURONIC ACID AND MAGNESIUM ASCORBYL PHOSPHATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.08.2012 FR 1258082**
**29.08.2012 US 201261694527 P**

(43) Date de publication de la demande:
**08.07.2015 Bulletin 2015/28**

(60) Demande divisionnaire:
**20206847.4 / 3 797 757**

(73) Titulaire: **Laboratoires Vivacy**
**75116 Paris (FR)**

(72) Inventeurs:
• **BON BETEMPS, Jérémie**
  **F-73410 Albens (FR)**
• **PIRON, Estelle**
  **73490 La Ravoire (FR)**

(74) Mandataire: **Tripoz, Inès**
  **Cabinet Tripoz**
  **Le Pôle Sud**
  **22 rue Seguin**
  **69002 Lyon (FR)**

(56) Documents cités:
  **WO-A1-2011/086458    WO-A1-2012/104419**
  **WO-A2-2009/014559**

  • **None**

EP 2 890 360 B1

**Description**

L'acide hyaluronique est utilisé depuis plus de quinze ans dans le domaine de l'esthétique, où il a prouvé son innocuité et son efficacité. A ce jour, sur le marché des gels de comblement à visée esthétique ou « fillers », les gels à base d'acide hyaluronique réticulé d'origine biofermentaire sont les produits les plus utilisés.

L'utilisation de l'acide hyaluronique d'origine biofermentaire dans les domaines tels que le comblement de rides, la viscosupplémentation, le traitement ophtalmique ou encore le traitement de l'incontinence urinaire est d'autant plus reconnue et appréciée que de par sa présence naturelle dans le corps humain, et plus particulièrement dans le derme, le liquide synovial et la cornée, les risques dus aux effets secondaires sont minimisés.

Pour améliorer ces formulations d'acide hyaluronique et/ou leur conférer des propriétés particulières, un certain nombre d'additifs peuvent être ajoutés à ces formulations.

L'un des inconvénients principal de l'addition d'additifs dans les formulations est la dégradation potentielle de leurs propriétés rhéologiques et/ou viscoélastiques ou de leur stabilité, soit directement lors de l'addition soit lors des phases de stérilisation, soit dans le temps par exemple lors du stockage.

L'acide ascorbique, connue également sous le nom de vitamine C, est un antioxydant puissant, impliqué dans de nombreux processus biologiques comme la synthèse du collagène et le métabolisme de certains acides aminés. Malheureusement la vitamine C est instable dans des solutions aqueuses, elle ne peut donc pas être utilisée dans des formulations d'acide hyaluronique qui sont formulées dans l'eau. Certains de ses dérivés, les sels de l'ester phosphate de l'acide ascorbique classiquement appelés sels d'ascorbylphosphate, sont eux stables en formulation aqueuse.

Ils peuvent donc être théoriquement incorporés dans des formulations d'acide hyaluronique.

La diméthylsulfone est un puissant anti-inflammatoire qui inhibe la réponse allergique causée par les xénobiotiques et est capable de protéger, à la fois les kératinocytes et les fibroblastes de l'action négative des rayons ultraviolets et inhibe l'action des cytokines.

L'incorporation de la diméthylsulfone est donc également intéressante à envisager dans des formulations, destinées au comblement de rides ou à la viscosupplémentation.

De nombreuses compositions comprenant de l'acide hyaluronique et un sel d'ascorbyl phosphate ont été décrites, notamment des compositions cosmétiques comme par exemple les compositions décrites dans la demande WO1996/19099 au nom de LVMH ou dans la demande GB 2 228 197 au nom de CHISSO Corporation, cependant ces compositions sont pour la plupart des compositions dans lesquelles l'acide hyaluronique n'est pas le constituant principal, le ratio entre la teneur en acide hyaluronique et la teneur en sel d'ascorbyl phosphate est généralement inférieur à 1. Elles sont en outre destinées à un usage topique et non pas en viscosupplémentation ou en comblement et de ce fait ne sont pas soumises à des opérations de stérilisation.

D'autres compositions sont également décrites dans WO2000/71094 au nom de AMWAY Corporation, qui présentent les mêmes caractéristiques que les formulations décrites dans les demandes citées ci-dessus et ces compositions si elles permettent de confirmer l'intérêt des sels d'ascorbyl phosphate, elles n'enseignent pas la possibilité de formuler des produits de comblement ou de viscosupplémentation dont le constituant principal est l'acide hyaluronique avec un sel d'ascorbyl phosphate.

En revanche, dans la demande WO2011/086458 au nom de ALLERGAN, des compositions destinées au comblement de rides ou à la viscosupplémentation soumises à une stérilisation sont décrites, et elles comprennent de l'acide hyaluronique et des dérivés de vitamine C tels que l'acide ascorbique 2-glucoside, l'ascorbyl phosphate de sodium, ou encore l'ascorbyl phosphate de magnésium, à une teneur comprise entre 0,6 et 2 % en poids par rapport au poids de la composition.

D'après le breveté, les compositions comprenant de l'acide ascorbique 2-glucoside, permettraient d'obtenir des formulations qui sont stables et dont la stabilité augmente, voir à l'exemple 6, lorsque la concentration en acide ascorbique 2-glucoside augmente.

Les formulations comprenant de l'ascorbyl phosphate de sodium à 2%, exemple 5, sont, elles, considérées comme stables.

En revanche, les compositions comprenant de l'ascorbyl phosphate de magnésium à une teneur de 2 % en poids par rapport au poids total de la composition sont décrites par le breveté, voir à l'exemple 4, comme n'étant pas stables après stérilisation par autoclavage à la vapeur mais la teneur en acide hyaluronique desdites compositions n'est pas divulguée, les ratios [HA]/[MAP] ne sont donc pas calculables et les résultats inexploitables et impossible à reproduire.

Dans la demande WO 2012/104419 au nom de Q-MED, des compositions injectables destinées au comblement des rides ou à la viscosupplémentation soumises à une stérilisation sont décrites. Elles comprennent de l'acide hyaluronique, un anesthésique local et un dérivé d'acide ascorbique tel que notamment l'ascorbyl phosphate de sodium, l'ascorbyl phosphate de magnésium, ou encore l'acide ascorbique 2-glucoside à une teneur comprise entre 0,01 et 1 mg/mL.

Dans ces compositions, la présence du dérivé d'acide ascorbique permet d'empêcher ou de diminuer les effets négatifs de l'anesthésique local sur la viscosité et/ou l'élasticité de la composition au cours de la stérilisation. En effet, cette demande de brevet décrit des augmentations importantes des viscosités et des composantes élastiques G' des compositions suite à l'ajout de l'anesthésique local.

Dans cette demande, il est précisé que les compositions comprenant un dérivé d'acide ascorbique ne présentent pas une stabilité améliorée par rapport aux même compositions ne comportant pas d'acide ascorbique, et que l'addition de dérivés d'acide ascorbique peut induire une légère diminution de la stabilité des compositions.

Des exemples montrent que l'addition d'ascorbyl phosphate de magnésium permet de contrer l'effet de la lidocaïne sur l'élasticité de compositions d'acide hyaluronique réticulé. Dans ce cas, l'élasticité de la composition est diminuée suite à l'ajout d'ascorbyl phosphate de magnésium.

D'autres exemples montrent que l'addition d'ascorbyl phosphate de magnésium permet de contrer l'effet de la lidocaïne sur la viscosité de compositions d'acide hyaluronique non réticulé. Dans ce cas, la viscosité de la composition est diminuée suite à l'ajout d'ascorbyl phosphate de magnésium.

Dans la demande WO 2009/005790 au nom de CARBYLAN BIOSURGERY, des compositions utilisées dans des formulations injectables ou implantables utilisées par exemple en viscosupplémentation ou des compositions adhésives pour applications biomédicales sont décrites. Ces compositions sont soumises à stérilisation et comprennent de l'acide hyaluronique modifié chimiquement par un substituant comportant une fonction thiol et de l'acide ascorbique ou un de ses dérivés tels que l'ascorbate de sodium ou l'ascorbate de palmitoyle.

Ces compositions comprenant de l'acide hyaluronique modifié présentent une stabilité améliorée.

Il a été mis en évidence de façon tout à fait surprenante et inattendue, compte tenu des enseignements de l'art antérieur ci-dessus décrits, que l'ascorbyl phosphate de magnésium, à des teneurs inférieures ou égales à 1 % dans des compositions comprenant de l'acide hyaluronique réticulé améliore ou conserve la caractéristique rhéologique de la composante élastique G' par rapport à des compositions comprenant de l'acide hyaluronique seul réticulé après stérilisation.

Comme cela est démontré dans les exemples, cet effet n'est pas observé pour les compositions comprenant de l'ascorbyl phosphate de sodium.

La présente invention permet ainsi d'obtenir des compositions comprenant de l'acide hyaluronique et un sel d'ascorbyl phosphate de magnésium dont les propriétés rhéologiques sont améliorées ou conservées après stérilisation.

Dans un mode de réalisation, lorsque l'acide hyaluronique est réticulé les compositions selon l'invention présentent des valeurs des caractéristiques rhéologiques améliorées après stérilisation, par rapport aux compositions ne comprenant pas de sel d'ascorbyl phosphate de magnésium.

La présente invention concerne une composition, comprenant au moins un acide hyaluronique ou l'un de ses sels, seuls ou en mélange, réticulé, et un sel d'ascorbyl phosphate, les procédés de fabrication de ladite composition et son utilisation pour la formulation d'une composition pour le comblement de rides ou d'une composition de viscosupplémentation.

La présente invention concerne une composition, stérilisée, par autoclavage à la vapeur comprenant au moins un acide hyaluronique réticulé ou l'un de ses sels biologiquement acceptables, seuls ou en mélange et de l'ascorbyl phosphate de magnésium à un ratio massique entre la teneur en acide hyaluronique ou l'un de ses sels [HA] et la teneur en ascorbyl phosphate de magnésium [MAP], [HA]/[MAP] supérieur ou égal à 1, la teneur en ascorbyl phosphate de magnésium étant comprise entre 0,001 et 1 % en poids par rapport au poids total de ladite composition dont la composante élastique G' est conservée ou améliorée après stérilisation et est comprise entre 5 et 400 Pa. L'acide hyaluronique réticulé ou l'un des ses sels biologiquement acceptables a un taux de réticulation compris entre 0,1 et 0,2. La masse molaire Mw dudit acide hyaluronique réticulé ou l'un des ses sels biologiquement acceptables est comprise entre 0,01 et 5 MDa.

Dans un mode de réalisation, la composante élastique G' est conservée ou améliorée après stérilisation et est comprise entre 5 et 350 Pa.

Dans un mode de réalisation, la composante élastique G' est conservée ou améliorée après stérilisation et est comprise entre 50 et 300 Pa.

La composition est stérilisée par autoclavage à la vapeur.

On entend par acide hyaluronique ou l'un de ses sels biologiquement acceptable, seuls ou en mélange, l'acide hyaluronique, réticulé, seul ou en mélange, éventuellement modifié chimiquement par substitution, seul ou en mélange.

Dans un mode de réalisation, la composition selon l'invention comprend en outre un acide hyaluronique non réticulé ou l'un de ses sels, seul ou en mélange.

Dans un mode de réalisation, la composition selon l'invention comprend au moins un acide hyaluronique réticulé ou l'un de ses sels, seul ou en mélange.

Dans un mode de réalisation, la composition selon l'invention comprend au moins un acide hyaluronique co-réticulé ou l'un de ses sels, seul ou en mélange.

Dans un mode de réalisation, la composition selon l'invention comprend au moins un acide hyaluronique modifié chimiquement par substitution ou l'un de ses sels, seul ou en mélange.

Dans un mode de réalisation, l'acide hyaluronique est sous forme de sel de sodium ou de potassium.

Dans un mode de réalisation, la composition selon l'invention est caractérisée en ce que le ratio massique entre la teneur en acide hyaluronique ou l'un de ses sels [HA] et la teneur en ascorbyl phosphate de magnésium [MAP], [HA]/[MAP] est compris entre 10 et 30.

Dans un mode de réalisation, la composition selon l'invention est caractérisée en ce que le ratio massique entre la teneur en acide hyaluronique ou l'un de ses sels [HA] et la teneur en ascorbyl phosphate de magnésium [MAP], [HA]/[MAP] est compris entre 15 et 25.

Dans un mode de réalisation, la composition selon l'invention est caractérisée en ce que le ratio massique entre la teneur en acide hyaluronique ou l'un de ses sels [HA] et la teneur en ascorbyl phosphate de magnésium [MAP], [HA]/[MAP] est environ égal à 20.

Dans un mode de réalisation, la composition selon l'invention est caractérisée en ce que la teneur en ascorbyl phosphate de magnésium est comprise entre 0,01 et 0,5 % en poids par rapport au poids total de la composition.

Dans un mode de réalisation, la composition selon l'invention est caractérisée en ce que la teneur en ascorbyl phosphate de magnésium est comprise entre 0,03 et 1 % en poids par rapport au poids total de la composition.

Dans un mode de réalisation, la composition selon l'invention est caractérisée en ce que la teneur en ascorbyl phosphate de magnésium est comprise entre 0,05 et 0,15 % en poids par rapport au poids total de la composition.

Dans un mode de réalisation, la composition selon l'invention est caractérisée en ce que la teneur en ascorbyl phosphate de magnésium est comprise entre 0,03 et 0,1 % en poids par rapport au poids total de la composition.

Dans un mode de réalisation, la composition selon l'invention est caractérisée en ce que la teneur en ascorbyl phosphate de magnésium est comprise entre 0,07 et 0,1% en poids par rapport au poids total de la composition.

Dans un mode de réalisation, la composition selon l'invention est caractérisée en ce que la teneur en ascorbyl phosphate de magnésium est comprise entre 0,01 et 10 mg/g de composition.

Dans un mode de réalisation, la composition selon l'invention est caractérisée en ce que la teneur en ascorbyl phosphate de magnésium est comprise entre 0,3 et 10 mg/g de composition.

Dans un mode de réalisation, la composition selon l'invention est caractérisée en ce que la teneur en ascorbyl phosphate de magnésium est comprise entre 0,1 et 5 mg/g de composition.

Dans un mode de réalisation, la composition selon l'invention est caractérisée en ce que la teneur en ascorbyl phosphate de magnésium est comprise entre 0,5 et 1,5 mg/g de composition.

Dans un mode de réalisation, la composition selon l'invention est caractérisée en ce que la teneur en ascorbyl phosphate de magnésium est comprise entre 0,3 et 1 mg/g de composition.

Dans un mode de réalisation, la composition selon l'invention est caractérisée en ce que la teneur en ascorbyl phosphate de magnésium est comprise entre 0,7 et 1 mg/g de composition.

On appelle Mw ou « masse moléculaire », la masse moléculaire moyenne en masse des polymères, mesurée en Daltons.

La composition selon l'invention est caractérisée en ce que la masse moléculaire Mw de l'acide hyaluronique ou l'un de ses sels est comprise dans un intervalle de 0,01 MDa et 5 MDa.

Dans un mode de réalisation, la composition selon l'invention est caractérisée en ce que la masse moléculaire Mw de l'acide hyaluronique ou l'un de ses sels est comprise dans un intervalle de 0,1 MDa et 3,5 MDa.

Dans un mode de réalisation, la composition selon l'invention est caractérisée en ce que la teneur totale en acide hyaluronique ou l'un de ses sels, est comprise entre 0,2 et 5% en poids par rapport au poids total de ladite composition.

Dans un mode de réalisation, la composition selon l'invention est caractérisée en ce que la teneur totale en acide hyaluronique ou l'un de ses sels est supérieure ou égale à 1 % en poids par rapport au poids total de ladite composition.

Dans un mode de réalisation, la composition selon l'invention est caractérisée en ce que la teneur totale en acide hyaluronique ou l'un de ses sels est comprise entre 2 mg/g et 50 mg/g de composition.

Dans un mode de réalisation, la composition selon l'invention est caractérisée en ce que la teneur totale en acide hyaluronique ou l'un de ses sels est comprise entre 4 mg/g et 40 mg/g de composition.

Dans un mode de réalisation, la composition selon l'invention est caractérisée en ce que la teneur totale en acide hyaluronique ou l'un de ses sels est comprise entre 5 mg/g et 30 mg/g de ladite composition.

Dans un mode de réalisation, la composition selon l'invention est caractérisée en ce que la teneur totale en acide hyaluronique ou l'un de ses sels est comprise entre 10 mg/g et 30 mg/g de ladite composition.

Dans un mode de réalisation, la composition selon l'invention est caractérisée en ce qu'elle comprend au moins un acide hyaluronique ou l'un de ses sels réticulé.

Dans la présente invention, le taux de réticulation X, est défini comme étant égal au rapport :

$$X = \frac{\text{(Nombre de moles de réticulant introduites dans le milieu réactionnel)}}{\text{(Nombre de moles de motif disacharidique introduites dans le milieu réactionnel)}}$$

L'acide hyaluronique réticulé présente un taux de réticulation X compris entre 0,1 et 0,2.

Dans un mode de réalisation, l'acide hyaluronique est co-réticulé tel que décrit dans la demande WO2000/046253.

Dans un mode de réalisation, la composition selon l'invention est caractérisée en ce qu'elle comprend un mélange d'acides hyaluroniques, ou l'un de leurs sels, réticulé et non réticulé.

Dans un mode de réalisation, la composition selon l'invention est caractérisée en ce qu'elle comprend un mélange d'acides hyaluroniques, ou l'un de leurs sels, réticulés.

Dans un mode de réalisation, le mélange d'acides hyaluroniques, ou l'un de leurs sels, réticulés est un mélange monophasique tel que celui décrit dans la demande de brevet WO2009/071697.

Dans un mode de réalisation, le mélange d'acides hyaluroniques, ou l'un de leurs sels, réticulés est un mélange obtenu par mélange de plusieurs acides hyaluroniques, ou l'un de leurs sels, de masses moléculaires différentes préalablement à leur réticulation, tel que décrit dans la demande WO2004/092222.

Dans un mode de réalisation, la composition selon l'invention est caractérisée en ce qu'elle comprend au moins un acide hyaluronique, ou l'un de ses sels, substitué par un groupement apportant des propriétés lipophile ou hydratante, comme par exemple les acides hyaluroniques substitués tels que décrits dans la demande FR 1161125 au nom de la demanderesse.

Dans un mode de réalisation, la composition comprend, en outre un autre polysaccharide.

Dans un mode de réalisation cet autre polysaccharide est choisi dans le groupe constitué par la cellulose, l'acide alginique ou l'un de leurs sels.

Dans un mode de réalisation, la composition selon l'invention est caractérisée en ce qu'elle comprend en outre de la diméthyl sulfone.

Dans un mode de réalisation, la composition selon l'invention est caractérisée en ce que la teneur en diméthyl sulfone est comprise entre 0,001 et 5 % en poids par rapport au poids total de ladite composition.

Dans un mode de réalisation, la composition selon l'invention est caractérisée en ce que la teneur en diméthyl sulfone est comprise entre 0,1 et 1 % en poids par rapport au poids total de ladite composition.

Dans un mode de réalisation, la composition selon l'invention est caractérisée en ce que la teneur en diméthyl sulfone est comprise entre 0,1 et 20 mg/g de composition.

Dans un mode de réalisation, la composition selon l'invention est caractérisée en ce que la teneur en diméthyl sulfone est comprise entre 1 et 10 mg/g de composition.

Dans un mode de réalisation, la composition selon l'invention est caractérisée en ce que ladite composition comprend en outre au moins un agent additionnel.

Dans un mode de réalisation, la composition selon l'invention est caractérisée en ce que l'agent additionnel est choisi parmi les antioxydants, les anesthésiques locaux, seuls ou en mélange.

Dans un mode de réalisation, la composition selon l'invention est caractérisée en ce que les anesthésiques locaux sont choisis dans le groupe constitué par la lidocaïne, la procaïne, la mépivacaïne, la ropivacaïne, la bupivacaïne, ou leurs sels pharmaceutiquement acceptables.

Dans un mode de réalisation, la composition selon l'invention est caractérisée en ce que l'anesthésique local est le chlorhydrate de lidocaïne.

Dans un mode de réalisation, la composition selon l'invention est caractérisée en ce que les antioxydants sont choisis parmi les polyols.

Dans un mode de réalisation, la composition selon l'invention est caractérisée en ce que les antioxydants sont choisis parmi le mannitol et le sorbitol, seul ou en mélange.

Dans un mode de réalisation, la composition selon l'invention est caractérisée en ce que l'agent antioxydant est le mannitol.

Dans un mode de réalisation, la composition selon l'invention est caractérisée en ce que l'agent antioxydant est le sorbitol.

Dans un mode de réalisation, la composition selon l'invention est caractérisée en ce que l'agent antioxydant est une mélange de mannitol et de sorbitol.

Dans un mode de réalisation, la composition selon l'invention est caractérisée en ce que la teneur en agent additionnel est comprise entre 0,01 et 10 % en poids par rapport au poids total de la composition.

Dans un mode de réalisation, la composition selon l'invention est caractérisée en ce que la teneur en agent additionnel est comprise entre 0,1 et 5 % en poids par rapport au poids total de la composition.

Dans un mode de réalisation, la composition selon l'invention est caractérisée en ce que la teneur en agent additionnel est comprise entre 0,1 et 100 mg/g de composition.

Dans un mode de réalisation, la composition selon l'invention est caractérisée en ce que la teneur en agent additionnel est comprise entre 1 et 50 mg/g de composition.

Dans un mode de réalisation, l'invention est une composition de viscosupplémentation caractérisée en ce qu'elle comprend au moins une composition selon l'invention.

Dans un mode de réalisation, l'invention est une composition pour le traitement de la xérophtalmie ou sécheresse oculaire, caractérisée en ce qu'elle comprend au moins une composition selon l'invention.

Selon les modes de réalisation, lesdites compositions sont utilisées en tant que larmes artificielles, gels lacrymaux ou lubrifiants.

La composition selon l'invention est caractérisée en ce que sa composante élastique G' est conservée ou améliorée après stérilisation et est comprise entre 5 et 400 Pa.

Dans un mode de réalisation, la composante élastique G' est conservé ou améliorée après stérilisation et est comprise entre 5 et 350 Pa.

Dans un mode de réalisation, la composante élastique G' est conservé ou améliorée après stérilisation et est comprise entre 50 et 300 Pa.

La demande décrit l'utilisation de l'ascorbyl phosphate de magnésium à une concentration comprise entre 0,3 et 10 mg/g pour conserver ou améliorer l'élasticité après stérilisation d'une composition comprenant au moins un acide hyaluronique ou l'un de ses sels biologiquement acceptables, seul ou en mélange.

Dans un mode de réalisation de l'utilisation, la teneur en acide hyaluronique ou l'un de ses sels, seuls ou en mélange, dans la composition est comprise entre 0,2 et 5 % en poids par rapport au poids total de ladite composition.

Dans un mode de réalisation de l'utilisation, la teneur en acide hyaluronique ou l'un de ses sels, seuls ou en mélange, dans la composition est supérieure ou égale à 1 % en poids par rapport au poids total de ladite composition.

Dans un mode de réalisation de l'utilisation, l'acide hyaluronique, ou l'un de ses sels est réticulé.

Dans un mode de réalisation de l'utilisation, la concentration en ascorbyl phosphate de magnésium dans la composition est comprise entre 0,3 et 10 mg/g, pour améliorer l'élasticité de ladite composition après stérilisation.

Dans un mode de réalisation de l'utilisation, la concentration en ascorbyl phosphate de magnésium dans la composition est comprise entre 0,7 et 3 mg/g.

Dans un mode de réalisation de l'utilisation, la concentration en ascorbyl phosphate de magnésium dans la composition est comprise entre 0,7 et 1 mg/g, pour améliorer l'élasticité de ladite composition après stérilisation.

La demande décrit également l'utilisation de l'ascorbyl phosphate de magnésium comme agent pour conserver ou améliorer l'élasticité après stérilisation d'une composition comprenant au moins un acide hyaluronique ou l'un de ses sels biologiquement acceptables, seul ou en mélange, la concentration en ascorbyl phosphate de magnésium étant comprise entre 0,3 et 10 mg/g, et le ratio massique entre la teneur en acide hyaluronique ou l'un de ses sels [HA] et la teneur en ascorbyl phosphate de magnésium [MAP], [HA]/[MAP] étant supérieur ou égal à 1.

La demande décrit également un procédé pour conserver ou améliorer l'élasticité après stérilisation d'une composition comprenant au moins un acide hyaluronique ou l'un de ses sels biologiquement acceptables, seul ou en mélange, consistant en l'ajout à la composition d'ascorbyl phosphate de magnésium à une concentration comprise entre 0,3 et 10 mg/g, et le ratio massique entre la teneur en acide hyaluronique ou l'un de ses sels [HA] et la teneur en ascorbyl phosphate de magnésium [MAP], [HA]/[MAP] étant supérieur ou égal à 1.

Dans un mode de réalisation du procédé et/ou de l'utilisation la masse moléculaire Mw de l'acide hyaluronique ou l'un de ses sels dans la composition est comprise entre 0,01 et 5 MDa.

Dans un mode de réalisation du procédé et/ou de l'utilisation la teneur en acide hyaluronique ou l'un de ses sels, seuls ou en mélange, dans la composition est comprise entre 0,2 et 5 % en poids par rapport au poids total de ladite composition.

Dans un mode de réalisation du procédé et/ou de l'utilisation la teneur en acide hyaluronique ou l'un de ses sels, seuls ou en mélange, dans la composition est supérieure ou égale à 1 % en poids par rapport au poids total de ladite composition.

Dans un mode de réalisation du procédé et/ou de l'utilisation la composition comprend de l'acide hyaluronique, ou l'un de ses sels, réticulé.

Dans un mode de réalisation du procédé et/ou de l'utilisation la concentration en ascorbyl phosphate de magnésium dans la composition est comprise entre 0,3 et 10 mg/g, pour améliorer l'élasticité de ladite composition après stérilisation.

Dans un mode de réalisation du procédé et/ou de l'utilisation la composition comprend en outre de l'acide hyaluronique, ou l'un de ses sels, non réticulé.

Dans un mode de réalisation du procédé et/ou de l'utilisation la concentration en ascorbyl phosphate de magnésium dans la composition est comprise entre 0,7 et 3 mg/g.

Dans un mode de réalisation du procédé et/ou de l'utilisation la concentration en ascorbyl phosphate de magnésium dans la composition est comprise entre 0,7 et 1 mg/g, pour améliorer l'élasticité de ladite composition après stérilisation.

L'invention concerne également un procédé de fabrication d'une composition selon l'invention.

Dans un mode de réalisation le procédé selon l'invention est caractérisé en ce qu'il comprend au moins :

- Une étape d'hydratation des fibres d'au moins un acide hyaluronique ou l'un de ses sels, seuls ou en mélange, pour obtenir un hydrogel, - une étape de réticulation à un taux de réticulation compris entre 0,1 et 0,2,
- Une étape de mélange d'une solution d'ascorbyl phosphate de magnésium avec l'hydrogel obtenu à l'étape précédente,
- Une étape d'homogénéisation, et
- Une étape de stérilisation par autoclavage à la vapeur.

Dans un mode de réalisation, le procédé selon l'invention est caractérisé en ce que l'étape d'hydratation est réalisée à température ambiante.

Dans un mode de réalisation, le procédé selon l'invention est caractérisé en ce que l'étape d'homogénéisation est réalisée à température ambiante.

Dans un mode de réalisation, le procédé selon l'invention est caractérisé en ce que l'étape de mélange d'une solution d'ascorbyl phosphate de magnésium avec l'hydrogel obtenu à l'étape d'hydratation est réalisée à une température adaptée au procédé de fabrication. Dans un mode de réalisation elle est réalisée à température ambiante.

L'étape de stérilisation est réalisée par autoclavage à la vapeur.

Dans un mode de réalisation, le procédé selon l'invention est caractérisé en ce que la stérilisation par autoclavage à la vapeur est réalisée à une température de 121 à 134°C, pendant une durée adaptée à la température.

Dans un mode de réalisation, le procédé selon l'invention est caractérisé en ce qu'il comprend en outre au moins une étape de conditionnement du mélange homogénéisé dans des seringues.

Dans un mode de réalisation, le procédé selon l'invention est caractérisé en ce qu'il comprend en outre au moins une étape de conditionnement du mélange homogénéisé dans des flacons uni-doses.

Dans un mode de réalisation, le procédé selon l'invention est caractérisé en ce que l'étape de réticulation est réalisée au moyen d'au moins un agent de réticulation.

Dans un mode de réalisation, le procédé selon l'invention est caractérisé en ce que l'agent de réticulation est bi- ou polyfonctionnel.

Dans un mode de réalisation, le procédé selon l'invention est caractérisé en ce que l'agent de réticulation bi- ou polyfonctionnel est choisi parmi le groupe constitué par l'éther d'éthylèneglycoldiglycidyle, l'éther de butanedioldiglycidyle (BDDE), l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde, l'épichlorohydrine ou bien encore le glutaraldéhyde, les carbodiimides tels que par exemple le 1-éthyl-3-[3-dimethylaminopropyl]carbodiimide hydrochloride (EDC).

Dans un mode de réalisation, le procédé selon l'invention est caractérisé en ce que l'agent de réticulation bifonctionnel est l'éther de butanedioldiglycidyle (BDDE) ou le 1,2,7,8-diépoxyoctane.

Dans un mode de réalisation, le procédé de fabrication selon l'invention est caractérisé en ce que l'étape de réticulation est mise en œuvre selon les techniques connues de l'homme du métier.

Dans un mode de réalisation, le procédé selon l'invention est caractérisé en ce qu'il comprend après l'étape de réticulation, au moins une étape de purification et lavage mise en œuvre selon les techniques connues de l'homme du métier.

Dans un mode de réalisation, le procédé selon l'invention est caractérisé en ce qu'il comprend en outre au moins une étape de mélange d'une solution de diméthyl sulfone avec l'hydrogel obtenu à l'étape d'hydratation.

Dans un mode de réalisation, le procédé selon l'invention est caractérisé en ce que l'étape de mélange d'une solution de diméthyl sulfone avec l'hydrogel obtenu à l'étape d'hydratation se situe avant l'étape d'homogénéisation.

Dans un mode de réalisation, le procédé selon l'invention est caractérisé en ce que l'étape de mélange d'une solution de diméthyl sulfone avec l'hydrogel obtenu à l'étape d'hydratation est réalisée à une température adaptée au procédé de fabrication. Dans un mode de réalisation elle est réalisée à température ambiante.

Dans un mode de réalisation, le procédé selon l'invention est caractérisé en ce qu'il comprend en outre au moins une étape de mélange d'une solution d'au moins un agent additionnel avec l'hydrogel obtenu à l'étape d'hydratation.

Dans un mode de réalisation, le procédé selon l'invention est caractérisé en ce que l'étape de mélange d'une solution d'au moins un agent additionnel avec l'hydrogel obtenu à l'étape d'hydratation se situe avant l'étape d'homogénéisation.

Dans un mode de réalisation, le procédé selon l'invention est caractérisé en ce que l'étape de mélange d'une solution d'au moins un agent additionnel avec l'hydrogel obtenu à l'étape d'hydratation est réalisée à une température adaptée au procédé de fabrication. Dans un mode de réalisation elle est réalisée à température ambiante.

L'invention concerne l'utilisation d'une composition selon l'invention, pour la formulation d'une composition pour le comblement de rides.

L'invention concerne l'utilisation d'une composition selon l'invention, pour la formulation d'une composition de viscosupplémentation.

Les applications visées sont plus particulièrement les applications communément répandues dans le cadre des viscoélastiques injectables et des polysaccharides utilisés ou potentiellement utilisables dans les pathologies ou traitements suivants :

- Injections esthétiques au niveau du visage : de comblement de rides, défauts cutanés ou volumatrices (pommettes, menton, lèvres) ;
- Injections volumatrices au niveau du corps : augmentation des seins et des fesses, augmentation du point G, vaginoplastie, reconstruction des lèvres vaginales, augmentation de la taille du pénis ;
- Traitement de l'arthrose, injection dans l'articulation en remplacement ou en complément du liquide synovial déficient ;
- Injection péri-urétrale pour le traitement de l'incontinence urinaire par insuffisance sphinctérienne ;
- Injection post-chirurgicale pour éviter les adhésions péritonéales notamment ;
- Injection suite à une chirurgie de la presbytie par incisions sclérales au laser ;
- Injection dans la cavité vitréenne ;
- Injection au cours de la chirurgie de la cataracte ;
- Injection dans les parties génitales.

Plus particulièrement, en chirurgie esthétique, en fonction de ses propriétés viscoélastiques et de rémanence, la composition obtenue selon le procédé de l'invention pourra être utilisée :

- pour le comblement des rides fines, moyennes ou profondes, et être injectée avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.

La composition selon l'invention trouve également une application importante en chirurgie articulaire et en chirurgie dentaire pour le comblement des poches parodontales par exemple.

Ces exemples d'utilisation ne sont nullement limitatifs, la composition selon la présente invention étant plus largement prévue pour :

- combler des volumes ;
- générer des espaces au sein de certains tissus, favorisant ainsi leur fonctionnement optimal ;
- remplacer des liquides physiologiques déficients.

L'invention concerne également un kit comprenant une composition selon l'invention, conditionnée en seringues et stérilisées après conditionnement.

La demande décrit également un kit comprenant une composition selon l'invention, conditionnée en flacons uni-doses et stérilisée après conditionnement.

La composition selon l'invention trouve également une application dans le domaine cosmétique ou pharmaceutique.

Plus particulièrement dans le domaine cosmétique, une composition selon l'invention sera utilisée comme ingrédient actif hydratant dans une composition cosmétique.

Dans le domaine pharmaceutique, une composition selon l'invention sera utilisée comme composition d'hydratation des yeux atteints de sécheresse oculaire, à savoir comme larme artificielle.

Dans ces applications cosmétiques et/ou pharmaceutiques, les compositions peuvent comprendre en outre tout ingrédient cosmétiquement ou pharmaceutiquement acceptable.

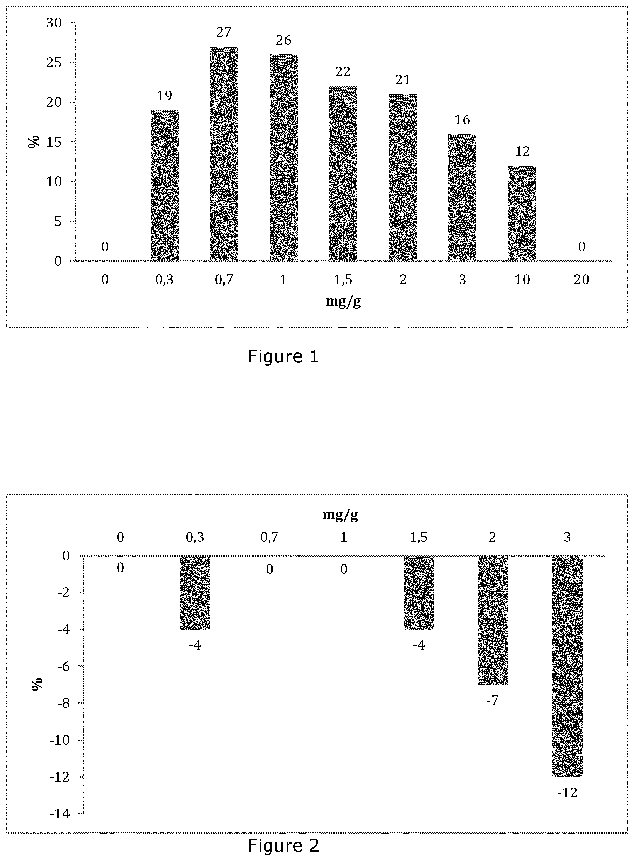

La figure 1 est un diagramme illustrant le pourcentage de gain, en ordonnée sur le diagramme, de la composante élastique G' en fonction de la teneur en ascorbyl phosphate de magnésium en mg/g de composition, en abscisse sur le diagramme, après stérilisation par autoclavage à la vapeur de compositions comprenant de l'acide hyaluronique réticulé et de l'ascorbyl phosphate de magnésium.

La figure 2 est un diagramme illustrant le pourcentage de gain, en ordonnée sur le diagramme, de la viscosité $\eta$ en fonction de la teneur en ascorbyl phosphate de magnésium en mg/g de composition, en abscisse sur le diagramme, après stérilisation par autoclavage à la vapeur de compositions comprenant de l'acide hyaluronique non réticulé et de l'ascorbyl phosphate de magnésium.

Les caractéristiques de la composition selon la présente invention ainsi que ses procédés de fabrication et leurs propriétés sont illustrés dans les exemples ci-après.

Exemple 1 hors de la portée de l'invention

Cet exemple illustre une composition comprenant de l'acide hyaluronique non réticulé et de l'ascorbyl phosphate de magnésium.

Des fibres de hyaluronate de sodium (NaHA) de qualité injectable (1 g ; masse moléculaire : environ 2,7 MDa) sont pesées dans un récipient. Une solution aqueuse de tampon phosphate (32,3 g) est ajoutée, le tout est homogénéisé pendant environ 1 heure à la spatule, à température ambiante et sous une pression atmosphérique de 900 mmHg.

L'hydrogel de NaHA non réticulé ainsi obtenu a une teneur d'environ 30 mg/g en NaHA.

De l'ascorbyl phosphate de magnésium (MAP) (60 mg soit $2,4. 10^{-4}$ mol) est solubilisé dans une solution de tampon phosphate (19,94 g) pour obtenir une solution aqueuse d'ascorbyl phosphate de magnésium de teneur 3 mg/g.

L'hydrogel de NaHA obtenu à l'étape précédente est dilué par ajout de la solution aqueuse d'ascorbyl phosphate de magnésium précédemment préparée. La composition ainsi obtenue est ensuite homogénéisée.

On obtient ainsi une composition comprenant du NaHA non réticulé à une teneur de 20 mg/g et de la MAP à une teneur de 1 mg/g ; le ratio massique [HA]/[MAP] est de 20.

La composition ainsi obtenue est conditionnée en seringues qui sont stérilisées par autoclavage à la vapeur (T= 121°C, 10 min).

Exemple 2

Cet exemple illustre un exemple de composition selon l'invention comprenant de l'acide hyaluronique réticulé et de l'ascorbyl phosphate de magnésium.

La composition comprenant de l'acide hyaluronique réticulé est obtenue selon le mode opératoire de réticulation décrit dans WO 2009/071697 (exemple 1, première partie) au nom de VIVACY à partir de fibres de hyaluronate de sodium (NaHA) (1 g ; masse moléculaire : environ 2,7 MDa) et de l'éther de butanedioldiglycidyle (BDDE) (54 mg). La composition ainsi obtenue a une teneur d'environ 30 mg/g en NaHA réticulé avec un taux de réticulation X d'environ 0,12.

Une solution aqueuse d'ascorbyl phosphate de magnésium de teneur 3 mg/g est préparée comme à l'exemple 1.

L'hydrogel de NaHA réticulé obtenu à l'étape précédente est dilué par ajout de la solution aqueuse d'ascorbyl phosphate de magnésium précédemment préparée. La composition ainsi obtenue est ensuite homogénéisée.

On obtient ainsi une composition comprenant du NaHA réticulé à une teneur de 20 mg/g et de l'ascorbyl phosphate de magnésium à une teneur de 1 mg/g ; le ratio massique [HA]/[MAP] est de 20.

La composition ainsi obtenue est conditionnée en seringues qui sont stérilisées par autoclavage à la vapeur (T=121°C, 10 min).

Exemple 3

Cet exemple illustre un exemple de composition selon l'invention comprenant de l'acide hyaluronique réticulé, de l'ascorbyl phosphate de magnésium et de la lidocaïne.

Une composition comprenant du NaHA réticulé est préparée selon le mode opératoire décrit à l'exemple 2 à partir d'un hydrogel de NaHA à une teneur de 30 mg/g et de l'ascorbyl phosphate de magnésium à une teneur de 10 mg/g.

L'addition d'une solution de lidocaïne à une teneur de 13 mg/g à la composition obtenue ci-dessus est effectuée selon le mode opératoire décrit dans WO 2009/024670 au nom de ANTEIS ou selon le mode opératoire décrit dans les demandes US 61/791,977 ou FR 13/52971 au nom de VIVACY.

La composition ainsi obtenue comprend de l'acide hyaluronique réticulé à une teneur de 20 mg/g, de la lidocaïne à une teneur de 3 mg/g et de l'ascorbyl phosphate de magnésium à une teneur de 1 mg/g ; le ratio massique [HA]/[MAP] est de 20.

La composition ainsi obtenue est conditionnée en seringues qui sont stérilisées par autoclavage à la vapeur (T=121°C, 10 min).

Exemple 4 hors de la portée de l'invention

Cet exemple illustre un exemple de composition comprenant de l'acide hyaluronique non réticulé, de l'ascorbyl phosphate de magnésium et de la diméthyl sulfone.

La composition comprenant de l'acide hyaluronique non réticulé et de l'ascorbyl phosphate de magnésium est préparée selon le mode opératoire de l'exemple 1, à partir d'un hydrogel d'acide hyaluronique à une teneur de 30 mg/g et d'une solution d'ascorbyl phosphate de magnésium à une teneur de 10 mg/g.

Une solution de diméthyl sulfone à une teneur de 4,3 mg/g est également ajoutée à la composition obtenue ci-dessus.

La composition ainsi obtenue est ensuite homogénéisée.

On obtient une composition qui comprend de l'acide hyaluronique non réticulé à une teneur de 20 mg/g, de la diméthyl sulfone à une teneur de 1 mg/g et de l'ascorbyl phosphate de magnésium à une teneur de 1 mg/g ; le ratio massique [HA]/[MAP] est de 20.

La composition ainsi obtenue est conditionnée en seringues qui sont stérilisées par autoclavage à la vapeur (T=121°C, 10 min).

Exemple 5

Cet exemple illustre un exemple de composition selon l'invention comprenant de l'acide hyaluronique réticulé, de l'ascorbyl phosphate de magnésium et de la diméthyl sulfone.

Une composition comprenant du NaHA réticulé est préparée selon le mode opératoire décrit à l'exemple 2 à partir d'un hydrogel de NaHA à une teneur de 30 mg/g et de l'ascorbyl phosphate de magnésium à une teneur de 10 mg/g.

Une solution de diméthyl sulfone à une teneur de 4,3 mg/g est également ajoutée à la composition obtenue ci-dessus.

La composition ainsi obtenue est ensuite homogénéisée.

On obtient une composition qui comprend de l'acide hyaluronique réticulé à une teneur de 20 mg/g, de la diméthyl sulfone à une teneur de 1 mg/g et de l'ascorbyl phosphate de magnésium à une teneur de 1 mg/g ; le ratio massique [HA]/[MAP] est de 20.

La composition ainsi obtenue est conditionnée en seringues qui sont stérilisées par autoclavage à la vapeur (T=121°C, 10 min).

Exemple 6 hors de la portée de l'invention

Cet exemple illustre un exemple de composition comprenant de l'acide hyaluronique non réticulé, de l'ascorbyl phosphate de magnésium et du mannitol.

Une composition comprenant du NaHA non réticulé est préparée selon le mode opératoire décrit à l'exemple 1 à partir d'un hydrogel de NaHA à une teneur de 30 mg/g et de l'ascorbyl phosphate de magnésium à une teneur de 10 mg/g.

Une solution de mannitol à une teneur de 4,3 mg/g est également ajoutée à la composition obtenue ci-dessus.

La composition ainsi obtenue est ensuite homogénéisée.

On obtient une composition qui comprend de l'acide hyaluronique non réticulé à une teneur de 20 mg/g, de l'ascorbyl phosphate de magnésium à une teneur de 1 mg/g et du mannitol à une teneur de 1 mg/g; le ratio massique [HA]/[MAP] est de 20.

La composition ainsi obtenue est conditionnée en seringues qui sont stérilisées par autoclavage à la vapeur (T=121°C, 10 min).

Exemple 7

Cet exemple illustre un exemple de composition comprenant de l'acide hyaluronique réticulé, de l'ascorbyl phosphate de magnésium et du mannitol.

Une composition comprenant du NaHA réticulé est préparée selon le mode opératoire décrit à l'exemple 2 à partir d'un hydrogel de NaHA à une teneur de 30 mg/g et de l'ascorbyl phosphate de magnésium à une teneur de 10 mg/g.

Une solution de mannitol à une teneur de 4,3 mg/g est également ajoutée à la composition obtenue ci-dessus.

La composition ainsi obtenue est ensuite homogénéisée.

On obtient une composition qui comprend de l'acide hyaluronique réticulé à une teneur de 20 mg/g, de l'ascorbyl phosphate de magnésium à une teneur de 1 mg/g et du mannitol à une teneur de 1 mg/g; le ratio massique [HA]/[MAP] est de 20.

La composition ainsi obtenue est conditionnée en seringues qui sont stérilisées par autoclavage à la vapeur (T=121°C, 10 min).

Exemple 8

Cet exemple illustre un exemple de composition comprenant de 'acide hyaluronique réticulé et de l'ascorbyl phosphate de sodium (SAP).

La composition comprenant de l'acide hyaluronique réticulé et de l'ascorbyl phosphate de sodium est préparée selon le mode opératoire de l'exemple 2, à partir d'un gel d'acide hyaluronique à une teneur de 30 mg/g et de l'ascorbyl phosphate de sodium à une teneur de 3 mg/g.

La composition ainsi obtenue comprend de l'acide hyaluronique réticulé à une teneur de 20 mg/g et de l'ascorbyl phosphate de sodium à une teneur de 1 mg/g ; le ratio massique [HA]/[SAP] est de 20.

La composition ainsi obtenue est conditionnée en seringues qui sont stérilisées par autoclavage à la vapeur (T=121°C, 10 min).

Exemple 9 hors de la portée de l'invention

Ce contre-exemple illustre un exemple de composition comprenant de l'acide hyaluronique non réticulé et du mannitol.

La composition comprenant de l'acide hyaluronique non réticulé et du mannitol (MAN) est préparée selon le mode opératoire de l'exemple 1, à partir d'un hydrogel d'acide hyaluronique à une teneur de 30 mg/g et d'une solution de mannitol à une teneur de 3 mg/g.

La composition ainsi obtenue comprend de l'acide hyaluronique non réticulé à une teneur de 20 mg/g et du mannitol à une teneur de 1 mg/g ; le ratio massique [HA]/[MAN] est de 20.

La composition ainsi obtenue est conditionnée en seringues qui sont stérilisées par autoclavage à la vapeur (T=121°C, 10 min).

Exemple 10

Ce contre-exemple illustre un exemple de composition comprenant de l'acide hyaluronique réticulé et du mannitol.

Une composition comprenant du NaHA réticulé est préparée selon le mode opératoire décrit à l'exemple 2 à partir d'un hydrogel de NaHA à une teneur de 30 mg/g et du mannitol à une teneur de 3 mg/g.

La composition ainsi obtenue comprend de l'acide hyaluronique réticulé à une teneur de 20 mg/g et du mannitol à une teneur de 1 mg/g ; le ratio massique [HA]/[MAN] est de 20.

La composition ainsi obtenue est conditionnée en seringues qui sont stérilisées par autoclavage à la vapeur (T=121°C, 10 min).

Exemple 11

Caractérisation des propriétés rhéologiques avant et après stérilisation par autoclavage à la vapeur des compositions comprenant de l'acide hyaluronique réticulé ci-dessus exemplifiées.

Les composantes élastiques G', des compositions comprenant de l'acide hyaluronique réticulé avant et après stérilisation par autoclavage à la vapeur ont été mesurées sur rhéomètre TA Instrument AR 2000 Ex, en oscillation à 25°C, les valeurs de la composante élastique G' étant relevées à une fréquence de 1 Hz.

Pour toutes les mesures, une composition de référence est formulée, en remplaçant la solution aqueuse d'ascorbyl phosphate de magnésium par une quantité équivalente de solution aqueuse de tampon phosphate.

Le pourcentage d'amélioration de la composante élastique G' est défini comme étant :

$$\% \text{ d'amélioration } G' = \frac{Y - Y'}{Y} \cdot 100$$

avec Y = Pourcentage de perte à la stérilisation de la composante élastique G' de la composition de référence et Y' = Pourcentage de perte à la stérilisation de la composante élastique G' de la composition testée.

Huit compositions comprenant de l'acide hyaluronique réticulé et de l'ascorbyl phosphate de magnésium à une teneur comprise entre 0,3 et 20 mg/g dans la composition sont préparées selon le mode opératoire décrit à l'exemple 2.

Trois compositions comprenant de l'acide hyaluronique réticulé et du mannitol à une teneur comprise entre 1 et 10 mg/g dans la composition sont préparées selon le mode opératoire décrit à l'exemple 10.

Deux compositions comprenant de l'acide hyaluronique réticulé et de l'ascorbyl phosphate de sodium à une teneur comprise entre 1 et 5 mg/g dans la composition sont préparées selon l'exemple 8.

Une composition de référence comprenant de l'acide hyaluronique réticulé est préparée selon le mode opératoire décrit à l'exemple 2. La solution aqueuse d'ascorbyl phosphate de magnésium est remplacée par une quantité équivalente d'une solution aqueuse de tampon phosphate.

Les composantes élastiques des compositions testées sont mesurées avant et après stérilisation et les pourcentages d'amélioration de la composante élastique G' sont calculés, les résultats obtenus sont présentés dans le tableau 1 ci-dessous :

Tableau 1

| Essai | [MAP] (mg/g) | Ratio [HA]/ [MAP] | [MAN] (mg/g) | [SAP] (mg/g) | % d'amélioration de la composante élastique G' par rapport à la composition de référence | Composante élastique G' (Pa) des gels stériles |
|---|---|---|---|---|---|---|
| 1 | 0 | n/a | 0 | 0 | 0 | 144 |
| 2 | 0,3 | 67 | 0 | 0 | 19 | 180 |
| 3 | 0,7 | 29 | 0 | 0 | 27 | 192 |
| 4 | 1 | 20 | 0 | 0 | 26 | 191 |
| 5 | 1,5 | 13 | 0 | 0 | 22 | 185 |
| 6 | 2 | 10 | 0 | 0 | 21 | 182 |
| 7 | 3 | 7 | 0 | 0 | 16 | 172 |
| 8 | 10 | 2 | 0 | 0 | 12 | 160 |
| 9 | 20 | 1 | 0 | 0 | 0 | 142 |
| 10 | 0 | n/a | 1 | 0 | 15 | 163 |
| 11 | 0 | n/a | 5 | 0 | 25 | 182 |
| 12 | 0 | n/a | 10 | 0 | 29 | 188 |
| 13 | 0 | n/a | 0 | 1 | -12 | 105 |
| 14 | 0 | n/a | 0 | 5 | -26 | 76 |
| n/a : non applicable | | | | | | |

Les résultats ci-dessus obtenus pour les compositions comprenant de la MAP sont également illustrés par la figure 1 qui est un diagramme représentant le pourcentage de gain de la composante élastique G' en fonction de la teneur en ascorbyl phosphate de magnésium après stérilisation par autoclavage à la vapeur de compositions comprenant de l'acide hyaluronique réticulé et de l'ascorbyl phosphate de magnésium.

On observe pour les compositions comprenant de l'acide hyaluronique réticulé et de l'ascorbyl phosphate de magnésium à une teneur comprise entre 0,3 et 20 mg/g que la composante élastique G' est améliorée, par rapport à la composition de référence, lorsque la teneur en ascorbyl phosphate de magnésium est comprise entre 0,3 et 10 mg/g avec une chute brutale lorsque cette teneur est supérieure à 10 mg/g.

Ces résultats sont en contradiction avec les résultats obtenus pour les compositions comprenant de l'acide hyaluronique réticulé et du mannitol qui montrent que plus on augmente la teneur en mannitol dans la composition, plus la composante élastique G' est améliorée par rapport à la composition de référence.

Ils sont également en contradiction avec les résultats obtenus pour les compositions comprenant de l'acide hyaluronique réticulé et de la SAP, pour lesquelles la composante élastique G' est diminuée aux deux concentrations testées.

Exemple 12 hors de la portée de l'invention

Caractérisation des propriétés rhéologiques avant et après stérilisation par autoclavage à la vapeur des compositions comprenant de l'acide hyaluronique non réticulé.

Les compositions comprenant de l'acide hyaluronique non réticulé sont caractérisées par leur viscosité η.

La viscosité η des compositions est mesurée sur rhéomètre TA Instruments AR 2000 Ex, en contrainte imposée à 25°C. La valeur de viscosité est relevée à une contrainte de 0,02 s-1.

Six compositions comprenant de l'acide hyaluronique non réticulé et de l'ascorbyl phosphate de magnésium à une teneur comprise entre 0,3 et 3 mg/g dans la composition sont préparées selon le mode opératoire décrit à l'exemple 1.

Une composition de référence comprenant de l'acide hyaluronique non réticulé est préparée selon le mode opératoire décrit à l'exemple 1. La solution aqueuse d'ascorbyl phosphate de magnésium décrite dans le mode opératoire à l'exemple 1 est remplacée par une quantité équivalente d'une solution aqueuse de tampon phosphate.

Le pourcentage d'amélioration de la viscosité $\eta$ est défini comme étant :

$$\% \text{ d'amélioration de la viscosité } \eta = \frac{Z-Z'}{Z} \cdot 100$$

avec Z = Pourcentage de perte à la stérilisation de la viscosité $\eta$ de la composition de référence
et Z' = Pourcentage de perte à la stérilisation de la viscosité $\eta$ de la composition testée.

Les viscosités des compositions testées sont mesurées avant et après stérilisation et les pourcentages d'amélioration de la viscosité sont calculés, les résultats obtenus sont présentés dans le tableau 2 ci-dessous :

Tableau 2

| Essai | [MAP] (mg/g) | % d'amélioration de la viscosité par rapport à la composition de référence |
|-------|--------------|---------------------------------------------------------------------------|
| 15 | 0 | 0 |
| 16 | 0,3 | -4 |
| 17 | 0,7 | 0 |
| 18 | 1 | 0 |
| 19 | 1,5 | -4 |
| 20 | 2 | -7 |
| 21 | 3 | -12 |

Les résultats ci-dessus obtenus sont également illustrés dans la figure 2 qui est un diagramme représentant le pourcentage de gain de la viscosité $\eta$ en fonction de la teneur en ascorbyl phosphate de magnésium après stérilisation par autoclavage à la vapeur de compositions comprenant de l'acide hyaluronique non réticulé et de l'ascorbyl phosphate de magnésium.

On observe une conservation de la viscosité pour les compositions selon l'invention ayant une teneur en ascorbyl phosphate de magnésium comprise entre 0,7 et 1 mg/g par rapport à la composition de référence.

Exemple 12bis a) hors de la portée de l'invention

Un même essai a été réalisé avec un suivi rhéologique de la composante élastique G'.

Sept compositions comprenant de l'acide hyaluronique non réticulé et de l'ascorbyl phosphate de magnésium à une teneur comprise entre 0,3 et 1,25 mg/g dans la composition sont préparées selon le mode opératoire décrit à l'exemple 1 (la masse moléculaire de l'hyaluronate de sodium est d'environ 2,7 MDa).

Une composition de référence comprenant de l'acide hyaluronique non réticulé est préparée selon le mode opératoire décrit à l'exemple 1. La solution aqueuse d'ascorbyl phosphate de magnésium décrite dans le mode opératoire à l'exemple 1 est remplacée par une quantité équivalente d'une solution aqueuse de tampon phosphate.

Les composantes élastiques G' des compositions testées sont mesurées avant et après stérilisation et les pourcentages d'amélioration de la composante élastique G' sont calculés, les résultats obtenus sont présentés dans le tableau 3 ci-dessous :

Tableau 3

| Essai | [MAP] (mg/g) | % d'amélioration de la composante élastique G' par rapport à la composition de référence | Composante élastique G' (Pa) |
|-------|--------------|------------------------------------------------------------------------------------------|------------------------------|
| 15 | 0 | 0 | 134 |
| 16 | 0,3 | 11 | 169 |
| 40 | 0,5 | 12 | 172 |

(suite)

| Essai | [MAP] (mg/g) | % d'amélioration de la composante élastique G' par rapport à la composition de référence | Composante élastique G' (Pa) |
|---|---|---|---|
| 17 | 0,7 | 7 | 161 |
| 41 | 0,85 | 5 | 148 |
| 18 | 1 | 4 | 153 |
| 42 | 1,25 | 0 | 132 |

On observe une amélioration de l'élasticité pour les compositions selon l'invention ayant une teneur en ascorbyl phosphate de magnésium comprise entre 0,3 et 1 mg/g par rapport à la composition de référence ainsi qu'une conservation de l'élasticité pour une teneur en ascorbyl phosphate de magnésium de 1,25 mg/g.

Exemple 12bis b) hors de la portée de l'invention

Un essai identique à l'essai 12 bis a) ci-dessus décrit a été réalisé avec des compositions comprenant du hyaluronate de sodium non réticulé de masse moléculaire d'environ 1,4 MDa et un suivi rhéologique de la composante élastique G'a été réalisé. Les compositions sont préparées selon le mode opératoire décrit à l'exemple 1 avec substitution du hyaluronate de sodium de masse moléculaire 2,7 MDa par le hyaluronate de sodium de masse moléculaire 1,4 MDa.
Les composantes élastiques G' des compositions testées sont mesurées avant et après stérilisation et les pourcentages d'amélioration de la composante élastique G' sont calculés, les résultats obtenus sont présentés dans le tableau 4 ci-dessous :

Tableau 4

| Essai | [MAP] (mg/g) | % d'amélioration de la composante élastique G' par rapport à la composition de référence | Composante élastique G' (Pa) |
|---|---|---|---|
| 43 | 0 | 0 | 7 |
| 44 | 0,7 | 20 | 10 |
| 45 | 1 | 16 | 9 |
| 46 | 3 | 0 | 7 |

On observe une amélioration de l'élasticité pour les compositions selon l'invention ayant une teneur en ascorbyl phosphate de magnésium comprise entre 0,7 et 1 mg/g par rapport à la composition de référence ainsi qu'une conservation de l'élasticité pour une teneur en ascorbyl phosphate de magnésium de 3 mg/g.

Exemple 13

Caractérisation des propriétés rhéologiques avant et après stérilisation par autoclavage à la vapeur de compositions comprenant de l'acide hyaluronique réticulé, de l'ascorbyl phosphate de magnésium et un agent additionnel.
Les propriétés rhéologiques avant et après stérilisation par autoclavage à la vapeur de compositions comprenant de l'acide hyaluronique réticulé, de l'ascorbyl phosphate de magnésium et un agent additionnel sont mesurées et les pourcentages d'amélioration de la composante élastique G' sont calculés, selon le protocole décrit à l'exemple 11.
Quatre compositions comprenant de l'acide hyaluronique réticulé, de l'ascorbyl phosphate de magnésium à une teneur de 1 mg/g et un agent additionnel à une teneur de 1 mg/g dans la composition sont préparées selon le mode opératoire décrit à l'exemple 7. Les agents additionnels exemplifiés sont le mannitol (MAN), le sorbitol (SOR), le sel hydrosoluble de sucrose octasulfate de potassium (KSOS) et du chlorhydrate de lidocaïne (LIDO).
Une composition comprenant de l'acide hyaluronique réticulé et de l'ascorbyl phosphate de magnésium à une teneur de 1 mg/g est préparée selon le mode opératoire décrit à l'exemple 2.
Une composition de référence comprenant de l'acide hyaluronique réticulé est préparée selon le mode opératoire décrit à l'exemple 2. La solution aqueuse d'ascorbyl phosphate de magnésium est remplacée par une quantité équivalente d'une solution aqueuse de tampon phosphate.
Les composantes élastiques des compositions testées sont mesurées avant et après stérilisation et les pourcentages d'amélioration de la composante élastique G' sont calculés, les résultats obtenus sont présentés dans le tableau 5 ci-dessous :

Tableau 5

| Essai | [MAP] (mg/g) | Agent additionnel à une teneur de 1 mg/g | % d'amélioration de la composante élastique G' par rapport à la composition de référence | Composante élastique G' (Pa) des gels stériles |
|---|---|---|---|---|
| 22 | 0 | 0 | 0 | 138 |
| 23 | 1 | 0 | 31 | 196 |
| 24 | 1 | MAN | 31 | 197 |
| 25 | 1 | SOR | 29 | 197 |
| 26 | 1 | KSOS | 29 | 195 |
| 27 | 1 | LIDO | 8 | 153 |

On observe que l'amélioration des propriétés rhéologiques observée à l'exemple 11 pour les compositions comprenant de l'acide hyaluronique et de l'ascorbyl phosphate de magnésium est conservée en présence de tous les agents additionnels. Seul, l'ajout de chlorhydrate de lidocaïne entraîne une diminution notable de cette amélioration.

Exemple 13bis

Caractérisation des propriétés rhéologiques avant et après stérilisation par autoclavage à la vapeur des compositions comprenant de l'acide hyaluronique réticulé, de l'ascorbyl phosphate de magnésium et de la Lidocaïne à 3 mg/g.

Les composantes élastiques des compositions testées sont mesurées avant et après stérilisation et les pourcentages d'amélioration de la composante élastique G' sont calculés, les résultats obtenus sont présentés dans le tableau 6 ci-dessous :

Tableau 6

| Essai | [MAP] (mg/g) | [LIDO] (mg/g) | % d'amélioration de la composante élastique G' par rapport à la composition de référence | Composante élastique G' (Pa) des gels stériles |
|---|---|---|---|---|
| 47 | 0 | 0 | 0 | 109 |
| 48 | 1 | 0 | 30 | 144 |
| 49 | 0 | 3 | -7 | 103 |
| 50 | 1 | 3 | -5 | 104 |
| 51 | 3 | 3 | 22 | 138 |
| 52 | 10 | 3 | 9 | 120 |

On observe que l'ajout de lidocaïne seule entraine une diminution de la composante élastique G' de la composition après stérilisation. L'ajout de MAP à 1 mg/g ne permet pas de contrer l'effet de la lidocaine sur l'élasticité de la composition. En revanche, l'ajout de MAP à 3 et 10 mg/g permet d'améliorer l'élasticité de la composition en présence de lidocaïne. Ces résultats sont inattendus et contraires aux enseignements et aux effets décrits dans l'art antérieur et notamment WO2012/104419 au nom de Q-MED ci-dessus cité.

Exemple 14 hors de la portée de l'invention

Caractérisation des propriétés rhéologiques avant et après stérilisation par autoclavage à la vapeur des compositions comprenant de l'acide hyaluronique non réticulé, de l'ascorbyl phosphate de magnésium et un agent additionnel.

Les propriétés rhéologiques avant et après stérilisation par autoclavage à la vapeur de compositions comprenant de l'acide hyaluronique non réticulé, de l'ascorbyl phosphate de magnésium et un agent additionnel sont mesurées et les pourcentages d'amélioration de la viscosité sont calculés, selon le protocole décrit à l'exemple 12.

Trois compositions comprenant de l'acide hyaluronique non réticulé, de l'ascorbyl phosphate de magnésium à une teneur de 1 mg/g et un agent additionnel à une teneur de 1 mg/g dans la composition sont préparées selon le mode opératoire décrit à l'exemple 6. Les agents additionnels exemplifiés sont le mannitol (MAN), le sorbitol (SOR) et le sel hydrosoluble de sucrose octasulfate de potassium (KSOS).

Une composition comprenant de l'acide hyaluronique non réticulé et de l'ascorbyl phosphate de magnésium à une teneur de 1 mg/g est préparée selon le mode opératoire décrit à l'exemple 1.

Une composition de référence comprenant de l'acide hyaluronique non réticulé est préparée selon le mode opératoire décrit à l'exemple 1. La solution aqueuse d'ascorbyl phosphate de magnésium décrite dans le mode opératoire à l'exemple 1 est remplacée par une quantité équivalente d'une solution aqueuse de tampon phosphate.

Les viscosités des compositions testées sont mesurées avant et après stérilisation et les pourcentages d'amélioration de la viscosité sont calculés, les résultats obtenus sont présentés dans le tableau 7 ci-dessous :

Tableau 7

| Essai | [MAP] (mg/g) | Agent additionnel à une teneur de 1 mg/g | % d'amélioration de la viscosité $\eta$ par rapport à la composition de référence |
|---|---|---|---|
| 28 | 0 | 0 | 0 |
| 29 | 1 | 0 | 0 |
| 30 | 1 | MAN | 0 |
| 31 | 1 | SOR | 0 |
| 32 | 1 | KSOS | 0 |

On observe une conservation de la viscosité pour toutes les compositions.

Exemple 15

Caractérisation des propriétés rhéologiques avant et après stérilisation par autoclavage à la vapeur des compositions comprenant de l'acide hyaluronique réticulé, de l'ascorbyl phosphate de magnésium et de la diméthyl sulfone (DMS).

Les propriétés rhéologiques avant et après stérilisation par autoclavage à la vapeur de compositions comprenant de l'acide hyaluronique réticulé, de l'ascorbyl phosphate de magnésium et de la diméthyl sulfone (DMS) sont mesurées et les pourcentages d'amélioration de la composante élastique G' sont calculés, selon le protocole décrit à l'exemple 11.

Deux compositions comprenant de l'acide hyaluronique réticulé, de l'ascorbyl phosphate de magnésium à une teneur de 1 mg/g dans la composition et de la diméthyl sulfone à une teneur comprise entre 1 et 10 mg/g dans la composition sont préparées selon le mode opératoire décrit à l'exemple 5.

Une composition comprenant de l'acide hyaluronique réticulé et de l'ascorbyl phosphate de magnésium à une teneur de 1 mg/g est préparée selon le mode opératoire décrit à l'exemple 2.

Une composition de référence comprenant de l'acide hyaluronique réticulé est préparée selon le mode opératoire décrit à l'exemple 2. La solution aqueuse d'ascorbyl phosphate de magnésium est remplacée par une quantité équivalente d'une solution aqueuse de tampon phosphate.

Les composantes élastiques des compositions testées sont mesurées avant et après stérilisation et les pourcentages d'amélioration de la composante élastique G' sont calculés, les résultats obtenus sont présentés dans le tableau 8 ci-dessous :

Tableau 8

| Essai | [MAP] (mg/g) | [DMS] (mg/g) | % d'amélioration de la composante élastique G' par rapport à la composition de référence | Composante élastique G' (Pa) des gels stériles |
|---|---|---|---|---|
| 33 | 0 | 0 | 0 | 185 |
| 34 | 1 | 0 | 29 | 243 |
| 35 | 1 | 1 | 30 | 242 |
| 36 | 1 | 10 | 29 | 246 |

On observe que l'amélioration des propriétés rhéologiques observée à l'exemple 11 pour les compositions comprenant de l'acide hyaluronique et de l'ascorbyl phosphate de magnésium est conservée en présence de DMS à une teneur comprise entre 1 et 10 mg/g.

Exemple 16 hors de la portée de l'invention

Caractérisation des propriétés rhéologiques avant et après stérilisation par autoclavage à la vapeur des compositions comprenant de l'acide hyaluronique non réticulé, de l'ascorbyl phosphate de magnésium et de la diméthyl sulfone (DMS).

Les propriétés rhéologiques avant et après stérilisation par autoclavage à la vapeur de compositions comprenant de l'acide hyaluronique non réticulé, de l'ascorbyl phosphate de magnésium et de la diméthyl sulfone (DMS) sont mesurées et les pourcentages d'amélioration de la viscosité sont calculés, selon le protocole décrit à l'exemple 12.

La composition comprenant de l'acide hyaluronique non réticulé, de l'ascorbyl phosphate de magnésium à une teneur de 1 mg/g dans la composition et de la diméthyl sulfone à une teneur de 1 mg/g dans la composition selon l'invention est préparée et stérilisée selon le mode opératoire décrit à l'exemple 4 et est caractérisée par rhéologie selon l'exemple 12.

Une composition comprenant de l'acide hyaluronique non réticulé et de l'ascorbyl phosphate de magnésium à une teneur de 1 mg/g est préparée selon le mode opératoire décrit à l'exemple 1.

Une composition de référence comprenant de l'acide hyaluronique non réticulé est préparée selon le mode opératoire décrit à l'exemple 1. La solution aqueuse d'ascorbyl phosphate de magnésium décrite dans le mode opératoire à l'exemple 1 est remplacée par une quantité équivalente d'une solution aqueuse de tampon phosphate.

Les viscosités des compositions testées sont mesurées avant et après stérilisation et les pourcentages d'amélioration de la viscosité sont calculés, les résultats obtenus sont présentés dans le tableau 9 ci-dessous :

Tableau 9

| Essai | [MAP] (mg/g) | [DMS] (mg/g) | % d'amélioration de la viscosité $\eta$ par rapport à la composition de référence |
|---|---|---|---|
| 37 | 0 | 0 | 0 |
| 38 | 1 | 0 | 0 |
| 39 | 1 | 1 | 0 |

On observe une conservation de la viscosité pour la composition comprenant de l'acide hyaluronique non réticulé, de l'ascorbyl phosphate de magnésium à une teneur de 1 mg/g et de la diméthyl sulfone à une teneur de 1 mg/g.

Exemple 17

Comparaison avec les compositions décrites dans l'art antérieur

Dans la demande internationale WO 2011/086458 au nom de ALLERGAN, l'exemple 4 décrit une composition comprenant de l'acide hyaluronique réticulé et de l'ascorbyl phosphate de magnésium à des teneurs comprises entre 0,6 et 2% en poids par rapport au poids total de la composition. D'après le breveté, ces compositions ne seraient pas stables car les valeurs de $\Delta$ Tan $\delta$ 1 Hz desdites compositions sont supérieures à 0,1.

Les valeurs de $\Delta$ Tan $\delta$ 1 Hz des compositions comprenant de l'acide hyaluronique réticulé et de l'ascorbyl phosphate de magnésium à des teneurs respectivement de 0,6% et de 1% sont non définies.

Seule la valeur de $\Delta$ Tan $\delta$ 1 Hz de la composition comprenant de l'acide hyaluronique réticulé et de l'ascorbyl phosphate de magnésium à une teneur de 2% est définie et est de 0,344, valeur supérieure à 0,1.

WO 2011/086458 enseigne également que plus la teneur en ascorbyl phosphate de magnésium est importante dans la composition, plus la valeur de $\Delta$ Tan $\delta$ 1 Hz dans ladite composition tend à diminuer et tend à se rapprocher de la valeur de 0,1.

Comme dans la demande ci-dessus citée, les compositions précédemment étudiées sont caractérisées par leur $\Delta$ Tan $\delta$ 1 Hz qui est la différence entre le Tan $\delta$ 1 Hz de la composition testée et le Tan $\delta$ 1 Hz de la composition de référence, Tan $\delta$ 1 Hz étant le ratio de la composante visqueuse G" sur la composante élastique G'.

Les valeurs de $\Delta$ Tan $\delta$ 1 Hz des compositions testées par rapport à la composition de référence sont présentées dans le tableau 7 ci-dessous :

Tableau 10

| Essai | [MAP] (mg/g) | [MAN] (mg/g) | [SAP] (mg/g) | $\Delta$ Tan $\delta$ 1 Hz |
|---|---|---|---|---|
| 1 | 0 | 0 | 0 | 0 |
| 2 | 0,3 | 0 | 0 | -0,0135 |

(suite)

| Essai | [MAP] (mg/g) | [MAN] (mg/g) | [SAP] (mg/g) | Δ Tan δ 1 Hz |
|---|---|---|---|---|
| 3 | 0,7 | 0 | 0 | -0,0145 |
| 4 | 1 | 0 | 0 | -0,0140 |
| 5 | 1,5 | 0 | 0 | -0,0128 |
| 6 | 2 | 0 | 0 | -0,0136 |
| 7 | 3 | 0 | 0 | -0,0084 |
| 8 | 10 | 0 | 0 | -0,0008 |
| 9 | 20 | 0 | 0 | 0,0240 |
| 10 | 0 | 1 | 0 | -0,0131 |
| 11 | 0 | 5 | 0 | -0,0196 |
| 12 | 0 | 10 | 0 | -0,0210 |
| 13 | 0 | 0 | 1 | 0,0203 |
| 14 | 0 | 0 | 5 | 0,0771 |

On observe que les valeurs de Δ Tan δ 1 Hz des compositions comprenant de l'acide hyaluronique réticulé et de l'ascorbyl phosphate de magnésium à une teneur comprise entre 0,3 et 20 mg/g sont inférieures à 0,1.

On peut également observer que les valeurs de Δ Tan δ 1 Hz des compositions comprenant de l'acide hyaluronique réticulé et du mannitol à une teneur comprise entre 1 et 10 mg/g sont inférieures à 0,1 ainsi que les valeurs de Δ Tan δ 1 Hz des compositions comprenant de l'acide hyaluronique réticulé et du SAP à une teneur comprise entre 1 et 5 mg/g.

En conclusion, les résultats obtenus pour les compositions comprenant de l'acide hyaluronique réticulé et de l'ascorbyl phosphate de magnésium comprises entre 0,3 et 20 mg/g sont en contradiction par rapport à l'enseignement de WO 2011/086458 car avec des teneurs en ascorbyl phosphate de magnésium équivalentes ou moindres à celle de WO 2011/086458 dans les compositions selon l'invention, les valeurs de Δ Tan δ 1 Hz desdites sont inférieures à 0,1.

Les mêmes déterminations des Δ Tan δ 1 Hz ont été effectuées sur des compositions comprenant de l'acide hyaluronique réticulé, de l'ascorbyl phosphate de magnésium et un agent additionnel et les valeurs obtenues sont présentées dans le tableau 8 ci-dessous :

Tableau 11

| Essai | [MAP] (mg/g) | Agent additionnel à une teneur de 1 mg/g | Δ Tan δ 1 Hz |
|---|---|---|---|
| 22 | 0 | 0 | 0 |
| 23 | 1 | 0 | - 0,0197 |
| 24 | 1 | MAN | - 0,0191 |
| 25 | 1 | SOR | - 0,0181 |
| 26 | 1 | KSOS | - 0,0142 |
| 27 | 1 | LIDO | - 0,0068 |

On observe comme précédemment que les valeurs de Δ Tan δ 1 Hz après stérilisation par autoclavage à la vapeur sont inférieures à 0,1.

Les mêmes déterminations des Δ Tan δ 1 Hz ont été effectuées sur des compositions comprenant de l'acide hyaluronique réticulé, de l'ascorbyl phosphate de magnésium et de la diméthyl sulfone (DMS) et les valeurs obtenues sont présentées dans le tableau 9 ci-dessous :

Tableau 12

| Essai | [MAP] (mg/g) | [DMS] (mg/g) | $\Delta$ Tan $\delta$ 1 Hz |
|---|---|---|---|
| 33 | 0 | 0 | 0 |
| 34 | 1 | 0 | -0,0050 |
| 35 | 1 | 1 | -0,0061 |
| 36 | 1 | 10 | -0,0064 |

On observe à nouveau que les valeurs de $\Delta$ Tan $\delta$ 1 Hz sont inférieures à 0,1.

**Revendications**

1. Composition, stérilisée par autoclavage à la vapeur, comprenant au moins un acide hyaluronique réticulé ou l'un de ses sels biologiquement acceptables, seuls ou en mélange et de l'ascorbyl phosphate de magnésium à un ratio massique entre la teneur en acide hyaluronique ou l'un de ses sels [HA] et la teneur en ascorbyl phosphate de magnésium [MAP], [HA]/[MAP] supérieur ou égal à 1, la teneur en ascorbyl phosphate de magnésium étant comprise entre 0,001 et 1 % en poids par rapport au poids total de ladite composition, **caractérisée en ce que** la composante élastique G' de ladite composition est conservée ou améliorée après stérilisation et est comprise entre 5 et 400 Pa, **en ce que** ledit au moins un acide hyaluronique réticulé ou l'un de ses sels biologiquement acceptables a un taux de réticulation compris entre 0,1 et 0,2, et **en ce que** la masse moléculaire Mw dudit acide hyaluronique ou de l'un de ses sels est comprise entre 0,01 et 5 MDa.

2. Composition selon la revendication 1, **caractérisée en ce que** la teneur en ascorbyl phosphate de magnésium est comprise entre 0,03 % et 1 % en poids par rapport au poids total de ladite composition.

3. Composition selon la revendication 1, **caractérisée en ce qu'**elle comprend en outre de la diméthyl sulfone.

4. Composition selon l'une quelconque des revendications précédentes, **caractérisée en ce que** la teneur en acide hyaluronique ou l'un de ses sels, seuls ou en mélange, est comprise entre 0,2 et 5 % en poids par rapport au poids total de ladite composition.

5. Composition selon l'une quelconque des revendications précédentes, **caractérisée en ce que** la teneur en acide hyaluronique ou l'un de ses sels, seuls ou en mélange, est supérieure ou égale à 1 % en poids par rapport au poids total de ladite composition.

6. Composition selon l'une quelconque des revendications précédentes, **caractérisée en ce que** ladite composition comprend en outre au moins un agent additionnel.

7. Composition selon la revendication 6, **caractérisée en ce que** l'agent additionnel est choisi parmi les antioxydants, les anesthésiques locaux, seuls ou en mélange.

8. Composition selon la revendication 7, **caractérisée en ce que** les anesthésiques locaux sont choisis dans le groupe constitué par la lidocaïne, la procaïne, la mépivacaïne, la ropivacaïne, la buvipacaïne, ou leurs sels pharmaceutiquement acceptables.

9. Composition selon la revendication 7, **caractérisée en ce que** les antioxydants sont choisis parmi les polyols.

10. Procédé de fabrication d'une composition selon les revendications 1 à 9, **caractérisé en ce que** ledit procédé de fabrication comprend au moins :

    - Une étape d'hydratation des fibres d'au moins un acide hyaluronique ou l'un de ses sels, seuls ou en mélange, pour obtenir un hydrogel,
    - Une étape de réticulation à un taux de réticulation compris entre 0,1 et 0,2,
    - Une étape de mélange d'une solution d'ascorbyl phosphate de magnésium avec l'hydrogel obtenu à l'étape précédente,

- Une étape d'homogénéisation, et
- Une étape de stérilisation par autoclavage à la vapeur.

**11.** Procédé de fabrication selon la revendication 10, **caractérisé en ce que** ledit procédé comprend en outre au moins une étape de mélange d'une solution de diméthyl sulfone avec l'hydrogel obtenu à l'étape d'hydratation des fibres d'au moins un acide hyaluronique ou l'un de ses sels, seuls ou en mélange.

**12.** Utilisation d'une composition selon les revendications 1 à 9, pour la formulation d'une composition pour le comblement de rides.

**13.** Utilisation d'une composition selon les revendications 1 à 9, pour la formulation d'une composition de viscosupplémentation.

**14.** Kit comprenant une composition selon les revendications 1 à 9, conditionnée en seringues stériles.

**Patentansprüche**

**1.** Durch Dampfautoklavieren sterilisierte Zusammenssetzung umfassend wenigstens eine vernetzte Hyaluronsäure oder eines ihrer biologisch akzeptablen Salze, alleine oder als Mischung, und Magnesiumascorbylphosphat mit einem Massenverhältnis aus dem Gehalt an Hyaluronsäure oder einem ihrer Salze [HA] und dem Gehalt an Magnesiumascorbylphosphat [MAP], [HA]/[MAP], von 1 oder mehr, wobei der Gehalt an Magnesiumascorbylphosphat zwischen 0,001 und 1 Gew.-% bezogen auf das Gesamtgewicht der Zusammensetzung beträgt, **dadurch gekennzeichnet, dass** die elastische Komponente G' der Zusammensetzung nach Sterilisation beibehalten oder verbessert ist und zwischen 5 und 400 Pa beträgt, dass die wenigstens eine vernetzte Hyaluronsäure oder eines ihrer biologisch akzeptablen Salze einen Vernetzungsgrad zwischen 0,1 und 0,2 aufweist, und dass das Molekulargewicht Mw der besagten Hyaluronsäure oder eines ihrer Salze zwischen 0,01 und 5 MDa beträgt.

**2.** Zusammensetzung nach Anspruch 1, **dadurch gekennzeichnet, dass** der Gehalt an Magnesiumascorbylphosphat zwischen 0,03 Gew.-% und 1 Gew.-% bezogen auf das Gesamtgewicht der Zusammensetzung beträgt.

**3.** Zusammensetzung nach Anspruch 1, **dadurch gekennzeichnet, dass** sie weiterhin Dimethylsulfon umfasst.

**4.** Zusammensetzung nach einem der vorangehenden Ansprüche, **dadurch gekennzeichnet, dass** der Gehalt an Hyaluronsäure oder einem ihrer Salze, alleine oder als Mischung, zwischen 0,2 und 5 Gew.-% bezogen auf das Gesamtgewicht der Zusammensetzung beträgt.

**5.** Zusammensetzung nach einem der vorangehenden Ansprüche, **dadurch gekennzeichnet, dass** der Gehalt an Hyaluronsäure oder einem ihrer Salze, alleine oder als Mischung, 1 Gew.-% oder mehr bezogen auf das Gesamtgewicht der Zusammensetzung beträgt.

**6.** Zusammensetzung nach einem der vorangehenden Ansprüche, **dadurch gekennzeichnet, dass** die Zusammensetzung weiterhin wenigstens ein zusätzliches Mittel umfasst.

**7.** Zusammensetzung nach Anspruch 6, **dadurch gekennzeichnet, dass** das zusätzliche Mittel ausgewählt ist aus Antioxidantien, Lokalanästhetika, alleine oder als Mischung.

**8.** Zusammensetzung nach Anspruch 7, **dadurch gekennzeichnet, dass** die Lokalanästhetika ausgewählt sind aus der Gruppe bestehend aus Lidocain, Procain, Mepivacain, Ropivacain, Buvipacain und ihren pharmazeutisch akzeptablen Salzen.

**9.** Zusammensetzung nach Anspruch 7, **dadurch gekennzeichnet, dass** die Antioxidantien ausgewählt sind aus Polyolen.

**10.** Verfahren zum Herstellen einer Zusammenssetzung nach einem der Ansprüche 1 bis 9, **dadurch gekennzeichnet, dass** das Verfahren zum Herstellen wenigstens umfasst:

- einen Hydratisierungsschrit von Fasern aus mindestens einer Hyaluronsäure oder einem ihrer Salze, alleine oder als Mischung, um ein Hydrogel zu erhalten,
- einen Vernetzungsschritt zu einem Vernetzungsgrad zwischen 0,1 und 0,2,
- einen Schritt aus Mischen einer Magnesiumascorbylphosphatlösung mit dem aus dem vorhergehenden Schritt erhaltenen Hydrogel,
- einen Homogenisierungsschritt, und
- einen Sterilisierungsschritt mittels Dampfautoklavieren.

**11.** Verfahren zum Herstellen nach Anspruch 10, **dadurch gekennzeichnet, dass** es zusätzlich wenigstens einen Schritt aus Mischen einer Dimethylsulfonlösung mit dem Hydrogel umfasst, das in dem Hydratisierungsschritt von Fasern aus wenigstens einer Hyaluronsäure oder einem ihrer Salze, alleine oder als Mischung, erhalten wird.

**12.** Verwendung einer Zusammensetzung nach einem der Ansprüche 1 bis 9 zum Formulieren einer Zusammensetzung zur Faltenfüllung.

**13.** Verwendung einer Zusammensetzung nach einem der Ansprüche 1 bis 9 zum Formulieren einer Zusammensetzung zur Viskositätssupplementierung.

**14.** Kit umfassend eine Zusammensetzung nach einem der Ansprüche 1 bis 9, verpackt in sterilisierten Spritzen.

**Claims**

**1.** A composition sterilized by steam autoclaving, comprising at least one crosslinked hyaluronic acid or one of biologically acceptable salts thereof, alone or in a mixture and magnesium ascorbyl phosphate, in a ratio [HA]/[MAP] by mass of the amount of hyaluronic acid or salt thereof [HA] to the amount of magnesium ascorbyl phosphate [MAP] of greater than or equal to 1, the amount of magnesium ascorbyl phosphate being between 0.001% and 1% by weight, relative to the total weight of said composition, wherein the elastic component G' of the said composition is retained or improved after sterilization and is comprised from 5 to 400 Pa and wherein the at least crosslinked hyaluronic acid or one of biologically acceptable salts thereof has a degree of crosslinking comprised from 0.1 to 0.2 and wherein the molecular mass Mw of the said hyaluronic acid or one salts thereof is between 0.01 and 5 MDa.

**2.** The composition as claimed in claim 1, wherein the amount of magnesium ascorbyl phosphate is comprised from 0.03% and 1% by weight, relative to the total weight of said composition.

**3.** The composition as claimed in claim 1, further comprising dimethyl sulfone.

**4.** The composition as claimed in any one of the preceding claims, wherein the amount of hyaluronic acid or one of salts thereof, alone or in a mixture, is comprised from 0.2% to 5% by weight, relative to the total weight of said composition.

**5.** The composition as claimed in any one of the preceding claims, wherein the amount of hyaluronic acid or one of salts thereof, alone or in a mixture, is greater than or equal to 1% by weight, relative to the total weight of said composition.

**6.** The composition as claimed in any one of the preceding claims, said composition further comprising at least one additional agent.

**7.** The composition as claimed in claim 6, wherein the additional agent is selected from antioxidants and local anesthetics, alone or in a mixture.

**8.** The composition as claimed in claim 7, wherein the local anesthetics are selected from the group consisting of lidocaine, procaine, mepivacaine, ropivacaine, bupivacaine, or pharmaceutically acceptable salts thereof.

**9.** The composition as claimed in claim 7, wherein the antioxidants are selected from polyols.

**10.** A method for producing a composition as claimed in claims 1 to 9, wherein said production method comprises at least:

- a step of hydrating fibers of at least one hyaluronic acid or one of salts thereof, alone or in a mixture, to give a hydrogel,
- a step of crosslinking to a degree of crosslinking comprised from 0.1 to 0.2,
- a step of mixing a solution of magnesium ascorbyl phosphate with the hydrogel obtained in the preceding step,
- a step of homogenizing; and
- a step of sterilizing by steam autoclaving.

11. The production method as claimed in claim 10, wherein said method further comprises at least one step of mixing a solution of dimethyl sulfone with the hydrogel obtained in the step of hydrating fibers of at least one hyaluronic acid or salt thereof, alone or in a mixture.

12. The use of a composition as claimed in claims 1 to 9 to formulate a composition for filling in wrinkles.

13. The use of a composition as claimed in claims 1 to 9 to formulate a composition for viscosupplementation.

14. A kit comprising a composition as claimed in claims 1 to 9, packaged in sterile syringes.

Figure 1

Figure 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 199619099 A
- GB 2228197 A
- WO 200071094 A
- WO 2011086458 A
- WO 2012104419 A
- WO 2009005790 A
- WO 2000046253 A
- WO 2009071697 A
- WO 2004092222 A
- FR 1161125
- WO 2009024670 A
- US 61791977 B
- FR 1352971